# EUROPEAN PATENT APPLICATION

(11) **EP 1 111 039 A1**
(43) Date of publication of application: **27.06.2001**
(21) Application number: 99870279.9
(22) Date of filing: 22.12.1999
(51) Int. Cl.: C12N 5/08, A61K 39/00, A61P 35/00

(54) **Immature autologous clinical grade dendritic cells intended for vaccination of cancer patients**

(71) Applicant: UNIVERSITE LIBRE DE BRUXELLES, B-1050 Bruxelles (BE)
(72) Inventor: Lambermont, Micheline, 1410 Brussels (BE); Toungouz-Nevessignsky, Michel, 1180 Brussels (BE)
(74) Representative: De Clercq, Ann

(57) **Abstract**

The present invention is related to a composition containing blood cells, characterised in that it comprises a percentage of dendritic cells higher than 30% and lower than 95% of the total number of blood cells present in the composition.

## Description

### Field of the invention

The present invention is related to a new composition comprising immature autologous clinical grade dendritic cells obtained from leukapheresis products in a closed system and intended for the vaccination of cancer patients as well as their generation method in a closed system.

### Background of the invention and state of the art

Dendritic cells (DC) are key players in the initiation of immune responses. This cell type constitutes the most potent antigen presenting cell endowed with the unique capacity to cluster naïve T cells (1). Consequently, it has been proposed as a natural adjuvant aiming at the triggering of T cell responses against poor immunogens such as tumour associated antigens (TAA) (2). The realisation of DC-based clinical trials has long been impaired by the low frequency of circulating DC available in the blood (1). The development of method able to generate large number of DC from hematopoïetic precursors has recently allowed the initiation of pioneer clinical trials. These methods use two main sources of DC precursors: CD34+ stem cells (3) and peripheral blood monocytes (4). The main constraints of generating DC from stem cells is that the culture time is long and that the obtaining of the CD34+ cells require mobilisation of the patient (5). All this is obviated by the use of monocytes as DC precursor because these cells normally present in the blood can differentiate into DC after a 7-day culture in presence of GM-CSF and IL-4 (4). Consequently, monocytes derived DC are the most commonly targeted DC type for vaccination protocols. These pilot trials have yielded promising results for cancer therapy (6,7,8). The final proof that this approach is clinically relevant will require the realisation of large scale multi-centric studies. Consequently, there is an urgent need to move from research practices toward new methods of DC generation in clinical grade conditions. These include the use of closed systems (culture bags with sterile connections), avoidance of exogenous proteins and respect of standard operating procedures (SOP) able to guaranty predefined specifications of the cellular product. The generation of DC in research conditions involves the use of research grade reagents namely medium containing non human proteins (fetal calf serum, FCS) and open culture procedures with standard culture plates. These conditions are clearly associated with unacceptable infectious risks. Moreover, the use of not standardised reagents such as FCS is a source of variability incompatible with large scale studies.

### Aims of the invention

The aim of the present invention is to provide a new composition comprising immature autologous clinical grade dendritic cells which are suitable for a vaccination of cancer patients.

Another aim of the present invention is to provide a simplified and GMP (Good Manufacture Practice) procedure of DC generation from leukapheresis products in closed system, by using synthetic culture media devoid of non-human proteins and which do not require the use of open separation steps (ficoll) and numerous culture plates as described in the state of the art.

### Summary of the invention

The present invention is related to a composition containing blood cells, preferably made of purified blood products comprising immature autologous clinical grade dendritic cells (which are advantageously obtained from leukapheresis products in a closed system) and which is able to induce an immune response in cancer patients. Said composition is a liquid medium with blood cells comprising a high percentage of dendritic cells, which means that the percentage of dendritic cells (compared to the other present blood cells) present in said blood product is higher than 30%, preferably higher than 50, 60 or 65%, more preferably higher than 70 or 75%, but lower than 95%, preferably lower than 90%, of the total number of blood cells present in the composition.

The other "cell contaminants" present in the composition are mainly (more than 50% of the remaining cells) lymphocytes, more specifically lymphocytes T (CD3+), few lymphocytes B (CD19+) and NK cells (CD16+/CD56+).

It should be understood that the other cellular liquid or solid components present in said composition are known products necessarily present in purified blood products obtained after use of material from various companies (for instance the leukapheresis pack obtained from Cobe, Denver, CO, USA).

This composition comprising immature autologous clinical grade dendritic cells can be directly administered to a patient or advantageously "activated" with known techniques, in order to boost the immune system by vaccination of cancer patients.

"A vaccination of cancer patients" is the possibility to induce an immune anti-tumour response in cancer patients, said immune response being preferably complete (humoral and cellular) in said patients suffering from carcinogenesis of tumoral development and metastases formation and being able to reduce this carcinogenesis, tumoral development or metastases formation.

Therefore, the cells of the composition according to the invention can be pulsed with known tumour-associated antigens (TAA) or can be the result of a fusion with tumoral cells as described in document W096/30030 incorporated herein by reference. Said fused cells can be dendritic-like cell/tumour cell hybrids and hybridomas, preferably a dendritic cell/tumour cell hybrid and hybridoma inducing an anti-tumour response.

Another aspect of the present invention is related to a pharmaceutical composition comprising an adequate pharmaceutical carrier or diluent and the active elements present in the composition according to the invention (the mixture of cells above-described) used as a medicament (for inducing an anti-tumour response).

The pharmaceutical composition according to the invention may also comprise a possible adjuvant having synergetic effects for inducing by the immune system an anti-tumour response or for inducing the possible side-effects of the active compound present in said pharmaceutical composition.

An adequate pharmaceutical carrier present in the pharmaceutical composition according to the invention can be any compatible non-toxic substance suitable for administering the composition according to the invention to a patient and obtain the desired therapeutical or prophylactic properties. The pharmaceutically acceptable carrier according to the invention suitable for oral administration are the ones well known by the person skilled in the art, such as tablets, coated or non-coated pills, capsules, solutions or syrups. Other adequate pharmaceutical carriers or vehicles may vary according to the mode of administration (cutaneous, epicutaneous, subcutaneous, intradermal, inhalation, patching, intravenous, intramuscular, parenteral, oral, etc.).

A further aspect of the present invention is related to the use of said pharmaceutical composition for the manufacture of a medicament intended for inducing an anti-tumour immune response in a patient, especially for the treatment and/or the prevention of carcinogenesis and/or cancer, especially tumour development and metastases formation.

Another aspect of the present invention is related to a method of treatment and/or prevention in a mammal patient (including human) of carcinogenesis and/or cancer, especially tumour development and metastases formation, said method comprising the step of administering a sufficient amount of the pharmaceutical composition according to the invention to said mammal (including human) for inducing an anti-tumour response against said carcinogenesis and/or said cancer.

A last aspect of the present invention is related to a method for the generation of immature autologous clinical grade dendritic cells intended for vaccination of cancer patients in a closed system (i.e. culture with one or more sterile connections). Said method comprises essentially the generation of immature autologous clinical grade dendritic cells in said closed system from leukapheresis products by using clinical grade cytokines or other molecules having similar properties upon the maturation and/or the differentiation of monocytes, preferably IL-4 and GM-CSF but also IL-13 and GM-CSF.

The obtained product comprising cells and containing mainly dendritic cells (which means that more than 30% of all the cells are dendritic cells, preferably more than 50% or more than 70%) is thereafter harvested for about 7 days culture (preferably for about 3 days to about 10 days culture), and thereafter loaded with one or more tumour-associated antigens (TAA) or fused with tumoral cells.

Preferably, the method according to the invention comprises the essential following steps of:
- obtaining from one or more patients a purified leukapheresis product,
- submitting this product to centrifugation at about 150 G (800 rpm) for about 15 min at room temperature in order to obtain a platelet rich plasma (PRP),
- submitting said platelet rich plasma (PRP) to one or more washing steps, preferably in PBS supplemented with 5% human albumin,
- recovering the obtained cell, possibly by centrifugation,
- adding to the cell a culture medium comprising X-VIVO 20,
- transferring said medium with said cell in a culture bag (preferably of the type ITX 1008 obtained from Baxter), in order to obtain an adherence of the monocytes for 1 hour at about 37 °C in a 5% CO₂ atmosphere,
- adding clinical grade cytokines, preferably suitable amounts of GM-CSF and IL-4 (final concentration 1000 U/ml) or other molecules having similar properties upon the maturation and differentiation of monocyte cells,
- recovering the obtained cells before their loading with relevant clinical grade tumour-associated antigens or before their fusion with tumoral cells as described in document WO96/30030 (incorporated herein by reference) in order to obtain dendritic-like cells / tumour cells hybrids and hybridomas for inducing an anti-tumour response, preferably dendritic cells / tumour cells hybrids or hybridomas.

The method according to the invention is particularly suitable for obtaining the composition according to the invention in a closed system. Said method can also be extrapolated for non-closed systems. However, said non-closed system may present the drawbacks of not being in optimal sterile conditions.

The present invention will be described in details in the following non-limiting examples in reference to the enclosed figures.

### Short description of the drawings

Figure 1 represents the phenotype of the generated DC.

Figure 2 represents tetanus toxoid (TT) and keyhole limpet haemocyanin (KLH) presentation to autologous T cells by fresh as compared to thawed DC (one representative experiment out of three). Values plotted are those obtained after subtraction of the background observed in wells containing autologous T cells and DC in the absence of either TT or KLH.

### EXAMPLES

### Example 1: Generation of immature autologous clinical grade dendritic cells

### Patients selection

27 cancer patients were enrolled between April 98 and June 99 in two protocols. The first protocol involved patients with tumour expressing either the MAGE-1 and/or -3 antigens. All patients were tumour free or beard only a minimal residual tumour mass detected either by CT-Scan or by PET-scan at the time of enrolment. The tumours included 13 melanomas, 2 colorectal cancer, 2 lung cancers, 1 head and neck cancer, 3 sarcomas, 1 bladder carcinoma, 1 glucagonoma and 1 adrenal cancer. All these tumours expressed the MAGE-1 and/or -3 TAA and the HLA-A1 and/or -A2 and/or B44 antigens. DC pulsed with the relevant MAGE derived peptide(s) were injected IV and SC. Three other patients were enrolled in a phase I/II trial of vaccination of prostate cancer. In this case, DC were not pulsed with any peptide. In this protocol, DC were injected directly in one involved lobe of the prostate through the rectum.

### Leukapheresis

As in the first protocol, the vaccination schedule included 4 series of DC injection, a maximum of 4 leukapheresis was performed for each patients. Some patients underwent less leukapheresis because of withdrawal from the study. In the second protocol, only one leukapheresis was performed and after DC generation, DC were splitted into three, the first third being used as fresh product whereas the two remaining thirds were kept frozen until use (one and two weeks later). A total of 84 leukapheresis was performed using the Cobe Spectra (Cobe, Denver, CO, USA) Cell separator.

Peripheral access was obtained in all patients. The mean time of the procedure was roughly one hour which was able to treat a mean of one blood volume leading to the obtaining of 6x10⁹ WBC (table I).

### DC generation

### PRP procedure

The leukapheresis product was transferred in one of the bag of a double transfer pack (Baxter Fenwal Division, Deerfield, USA). All cell transfers were performed after sterile connections (Terumo Sterile Tubing Welder, Terumo, Leuven, Belgium). After centrifugation at 150g (800 rpm) for 15 minutes at room temperature, the platelet rich plasma (PRP) was transferred in the other bag of the transfer pack using a plasma extractor and discarded.

### Washing

The cell containing bag was connected to a bag containing 375 ml of PBS (Baxter Fenwal Division, Deerfield, USA) supplemented with 5% human albumin (Human albumin 20%, DCF, Belgian Red Cross, Brussels, Belgium) in order to pour this solution onto the cells. The cells were then centrifuged for 15 min. at 150 g. The supernatant was discarded and the previous procedure was again performed in order to ensure a second washing.

### Seeding

2.25x10⁹ PBMC were transferred in one culture bag of 3 L capacity (ITX1008, Nexell Therapeutics, Irvine, CA, USA). X-Vivo 20 (Bio-Whittaker, Walkersville, MD, USA) was then added through a transfer pack to each culture bag in order to obtain a final volume of 450 ml. Monocytes present were then allowed to adhere by a 1 hour incubation at 37 °C in a 5% CO₂ atmosphere. After this adherence step, the supernatant was removed using a 50 ml syringe in order to discard as far as possible non adherent cells. 90 ml of X-Vivo 20 were subsequently added together with 5 ml of GM-CSF (final concentration: 800 U/ml) (Leukomax, Novartis, Basel, Switzerland) and 0.5 ml of IL-4 (final concentration: 1000 U/ml) (Schering-Plough, Kenilworth, NJ, USA). Bags were then placed in CO₂ incubators. Cells were fed every 2 days with GM-CSF and IL-4 at the same concentration.

A total number of 84 procedures was performed. 2.25x10⁹ PBMC were seeded each time yielding an mean number of 66x10⁶ DC (table II). The yield was calculated according to the absolute number of monocytes present in the starting cell product. It reached 12% on average. The viability of the DC was always higher than 98%. About two thirds of the cell products was represented by DC as assessed by morphological examination after May-Grünwald staining. The remaining cells were mostly represented by T cells (CD3+) whereas very few B cells (CD19+) or NK cells (CD16+CD56+) were present.

### Immunophenotype of the DC generated

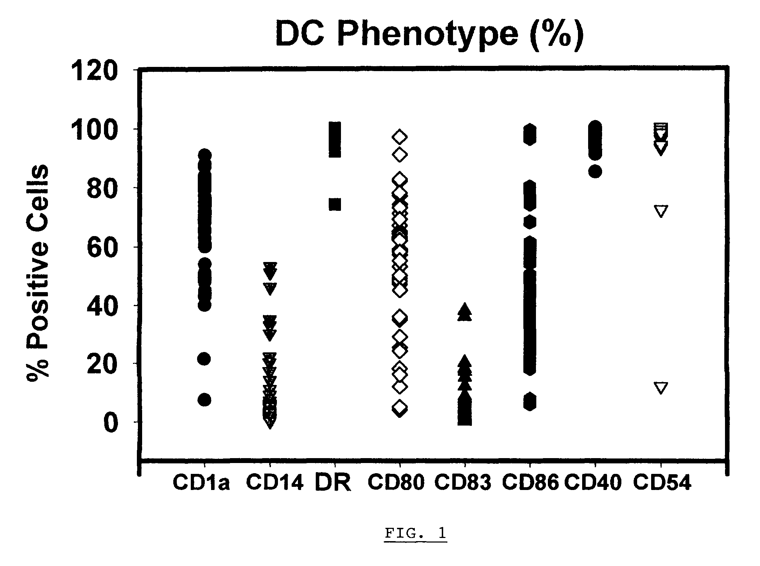

The DC obtained after 7 days of culture were CDla + (mean+/-SD: 65+/-20%), HLA-DR+ (98+/-3%), CD40+ (97+/-3%), CD54+ (97+/-11%). These cells were almost negative for CD14 (10+/-15%) and for CD83 (7+/-7%). About half of the cells were positive for the co-stimulatory molecules B7-1 (CD80: 49+/-22%) and B7-2 (CD86: 54+/-24%) (Fig. 1). This phenotype fits the characteristics displayed by immature DC.

### DC collection

After 7 days of culture, DC which are present in the supernatant were collected in 50 ml tubes (Falcon, Becton Dickinson, San Jose, CA, USA) and washed once in X-VIVO 20. Pellets containing the DC were suspended in X-VIVO and pooled in two 50 ml tube. They were again washed. Cells were numerated and viability was assessed by exclusion of trypan blue.

### DC Pulsing

DC concentration was adjusted to 2 millions cells/ml of X-Vivo 20 supplemented with autologous plasma (10%). Then, the relevant clinical grade of TAA peptides (obtained from UCB, Brussels, Belgium), MAGE-3.A1 (EVDPIGHLY) and/or MAGE-1.A1 (EADPTGHSY) and/or MAGE-3.A2 (FLWGPRALV) and/or MAGE-3.B44 (MEVDPIGHLY): were added at a concentration of 50 *µ*g/ml. After a 2 hours incubation at 37 °C in a 5% CO₂ atmosphere, cells were washed once in PBS Albumin 5% and suspended in 4 ml of this solution. The cells transferred into syringes were then ready to use.

### DC characterisation and quality control (QC)

### Morphology

DC were analysed by morphological examination after May-Grünwald-Giemsa staining under light microscopy.

### Viablity

The viability of the generated DC was assessed by trypan blue exclusion.

### Immunophenotype

The immunophenotype of the DC was performed by flow cytometry analysis after staining with the following monoclonal antibodies: anti-HLA-DR, -CD54, -CD14, -CD40, -CD80 (Ab from Becton Dickinson, San Jose, CA, USA), -CD86, -CD83 (Ab from Immunotech, Marseille, France) and -CD1a (Ab from Dako, Glostrup, Denmark). expression. Events acquisition was performed using a FACSScan (Becton-Dickinson, San Jose, CA, USA) on CD45+ cells (leukogate). Analysis was performed using the Cellquest software (Becton-Dickinson, San Jose, CA, USA) after morphological gating on DC according to forward scatter (FSC) and side scatter (SSC).

### Microbiology

After pulsing, DC were tested for the presence of bacteria and fungi by culture of the cell suspension on solid blood containing nutritive medium. Mycoplasma (Boerhinger Mannheim, Germany), and endotoxin detections (QCL-1000, Bio-Whittaker, Walkersville, MD, USA) were performed on the supernatant of the 7 day culture. Direct gram staining was performed on the final cell suspension just before injection.

All microbiological tests were negative.

### Freezing procedure

A cell suspension containing from 50 millions to 87 millions DC in 5m1 of PBS was poured onto the same volume of a freezing medium composed of PBS (45%), DMSO (15%) and 20% albumin (40%). The final product was frozen at -80 °C.

### Functional testing

DC were tested for their capacity to present tetanus toxoid (TT, Sigma, St-Louis, MO, USA) (400 ng/ml) and KLH (50 *µ* g/ml) (Vacmun, Biosyn, Mannheim, Germany). DC were cultured with autologous T cell at a 1:30 ratio in X-Vivo 20 for 24 hours. After this preincubation step, IFN-γ secretion was assessed by ELISPOT. Briefly, cells were transferred onto nitrocellulose membranes (Millipore, Bedford, MA, USA) previously coated with a capture anti-IFN-γ MoAb (MabTech, Nacka, Sweden). Cytokines secretion was revealed using a biotinylated anti-IFN-y MoAb (MabTech, Nacka, Sweden), extravidin peroxydase (Sigma, St Louis, MO, USA) and aminoethylcarabazole (AEC) (Sigma, St Louis, MO, USA) as substrate. Enumeration was performed by counting using light miscroscopy.

### Influence of freezing and thawing on the phenotype and function of the DC

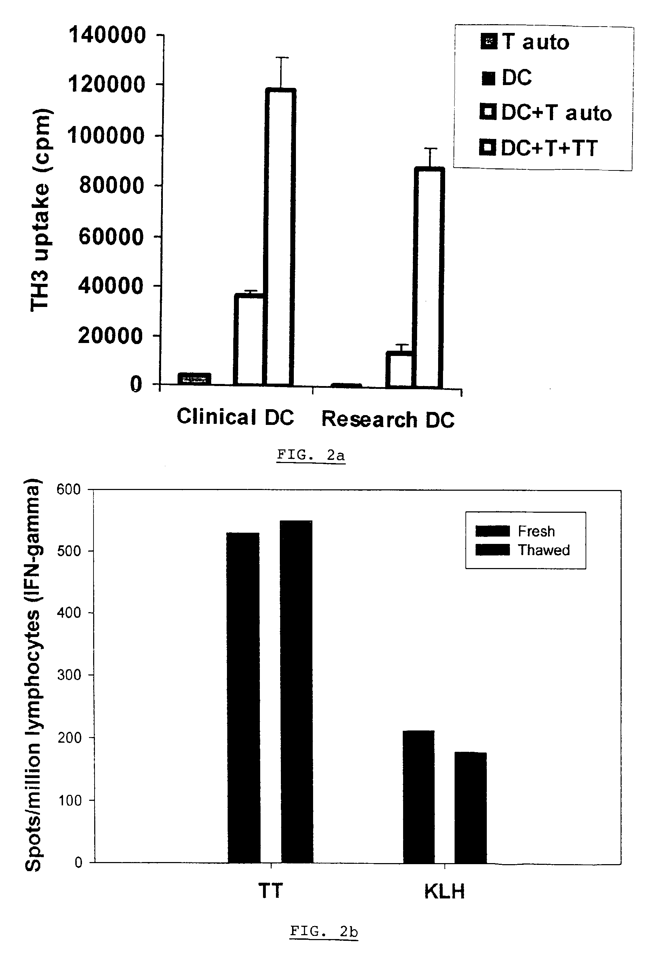

In our second protocol aimed at immunising prostate cancer patients, the inventors have set-up a freezing procedure of the generated DC in order to avoid repeated leukapheresis. The freezing medium used was the same as the one currently used in our institution for freezing of CD34+ selected stem cell products. The final products submitted to cryopreservation contained 7.5% DMSO in the presence of human albumin. This procedure was always associated with an excellent viability (>90%) of the thawed cells (table III). Moreover, their immunophenotype (table III as well as their functionality as assessed by tetanus toxoid and KLH presentations (figure 2) were not altered.

These data show that large number of functional immature dendritic cells can be reproducibly generated in cancer patients in a simple closed system using synthetic media devoid of non-human proteins and that this procedure is clinically safe.

The simplicity of the procedure was considerably improved as compared to previously described methods which imply an open separation step (ficoll) and the use of numerous culture plates (9). The generation procedure in itself was extremely safe. The leukapheresis product was obtained in about one hour in all patients using peripheral venous access obviating the need for repeated blood drawings. The further development of a freezing method able to preserve DC viability, phenotype and functionality even reduced the several leukapheresis initially required to one. On a microbiological standpoint, the procedure was extremely safe as the quality control remains always negative for all the infectious agents tested. These results are favoured by the strict use of a closed system during the 7-day culture limiting the manipulations and suppressing the direct exposition of cells to ambient air. This was possible thanks to the use of a peculiar plastic bag (Nexell ITX 1008) combined to a modified adherence step: one hour adherence followed by removal of non adherent cell without further washing. The result is a cellular product greatly enriched in DC. However, at the end of the generation procedure, it still remains about one third of "contaminating cells". These cells are mainly represented by T cells. In the present experience, 67% of the immunised patients (12 out of 18 assessable patients) disclosed an increase in the frequency of T cells secreting IFN-γ in response to the relevant tumour peptide indicating that the cellular vaccine was effective. What is also possibly occurring is that T cell partially activated during the generation procedure activate in some way DC through CD40L/D40 interaction (10). This interaction could be beneficial in stabilising DC in a DC state. Indeed it has been reported that immature DC trends to return to a macrophage state in the absence of continuous stimulation with GM-CSF and IL-4 (4). Whether DC must be mature or not for their use in anti-tumour vaccination is still controversial. Currently reported clinical trials demonstrating biological and/or clinical responses have mostly been used immature DC (6,7,8). However, induction of DC maturation in vitro is appealing as mature DC are stable and express higher levels of HLA and co-stimulatory molecules (11). However, some caution must be added to this view keeping in mind the example of bacterial lipopolysaccharide (LPS). LPS is a potent maturation agent. During LPS induced maturation, DC produce high amounts of IL-12, a pivotal cytokine for the differentiation of CD4+ T cells into Thl cells. However, presence of low doses of LPS at the initiation of DC culture induces a state of unresponsiveness characterised by the inhibition of their capacity to produce IL-12 and TNF-a on LPS re-challenge (12). This suggest that it could be more clinically relevant to preserve the possibility to achieve in vivo DC maturation through vaccination with immature DC in order to induce full in vivo T cell activation into the draining lymph nodes notably through efficient IL-12 production.

The simple method according to the invention is able to generate large number of DC in closed system using serum free medium from leukapheresis products. The functionality of this cellular product defined by its capacity to present antigens to autologous T cells and to safely immunise cancer patients against tumour associated antigens can be extrapolated to large scales studies under optimised safety conditions.

### Example 2: Generation of DLC hybrids and hybridomas

Female DBA/2 (H-2^{d}) and CBA/J (H-2^{k}), 6-8 week old, were purchased from Charles River Wiga (Sulzfeld, Germany) and maintained in a pathogen-free facility.

A tumor cell line (methylcholanthrene-induced mastocytoma P815 of DBA/2 origin, derived from a 6-thioguanine-resistant mutant) is prepared according to a described procedure (13). Briefly, P815 cells were cultured in DMEM supplemented with 10% FCS and increasing concentrations of 6-thioguanine (Sigma, St. Louis, MO), ranging from 1 to 30*µ*g/ml. The final 6-thioguanine-resistant cells died in HAT-medium, i.e. in medium supplemented with 10⁻⁴ M hypoxanthine (Merck, AG, Darmstadt, FRG), 3,8 10⁻⁷ M aminopterin (ICN Nutritional Chemicals) and 1.6x10⁻⁵ M 2-deoxythymidine (Merck AG). L1210 is a lymphocytic leukemia which arose in a DBA/2 female following painting the skin with methylcholanthrene (available through ATCC).

The generated composition according to example 1 comprising 2x10⁶ DC were mixed with 2x10⁶ HAT-sensitive P815 cells in a 15 ml conical tube. The cells were washed in RPMI 1640 and pelleted by centrifugation. The fusion was started by adding dropwise, in 90 seconds, 200 *µ*l of a 50% solution of PEG 4000 (Merck) in RPMI 1640 medium. The fusion was stopped by the stepwise addition of RPMI medium. The cells were centrifuged, resuspended in medium containing 10% FCS and additives, and incubated for 2 h, at 37 °C in 7% C0₂. The cells were centrifuged, resuspended in selection medium (RPMI 1640 containing HAT, 10% FCS and additives), and plated at 10⁴ cells/well in flat-bottomed 96-well plates (Becton Dickinson, CA, USA). The plates were seeded 1 day before use with a feeder layer consisting of 5,000 (irradiated peritoneal cells/well. The plated fusion was cultured at 37 °C in a 7% CO₂ atmosphere. The medium was renewed as required by cell growth.

The great majority of tumor antigens are either unknown or indeterminate with regard to their immunogenic T-cell epitopes. Furthermore, the method and composition of the invention combine several advantages such as the presence of costimulatory molecules, the ability to present antigen through the exogenous (MHC class II) and endogenous (MHC class I) pathways independently from known MHC/epitope associations. Presentation of multiple antigen derived epitopes may enhance anti-tumor immunity and minimize the emergence of resistant variants.

### In vitro response

The complete medium used in all experiments was RPMI 1640 (Seromed Biochem KG, Berlin, Germany) or DMEM (Gibco BRL, Merelbeke, Belgium) supplemented with 10% FCS, 2% ultroser HY (a serum-free medium supplement purchased from Gibco BRL) or 1% heat-inactivated mouse serum, penicillin, streptomycin, non-essential aminoacids, sodium pyruvate, 2-ME, and L-glutamine (Flow ICN Biomedicals, Bucks, UK).

### Mixed lymphocytes reaction (MLR)

Splenic CD4⁺ T-cells (CBA/J, H-2^{k}) were purified by depletion of adherent cells by passage over Sephadex G10 (Pharmacia Bioprocess, Uppsala, Sweden) and complement-mediated lysis with a cocktail of anti-B220 and anti-CD8 mAbs. 2x10⁵ CD4⁺ T-cells were stimulated with increasing numbers of γ-irradiated (15,000 rads) allogeneic P815 or hybrid cells, or with γ-irradiated (3000 rads) bone marrow-derived DC. Proliferation was assessed by thymidine incorporation during the last 16 h of a 4 day-culture. The supernatants were collected after 48 h of culture, frozen and assayed for IL-2 content using a standard bioassay with an IL-2 sensitive, IL-4 insensitive subclone of the CTL.L line.

### Tumor specific immune response

Resistant mice (injected with live P815 and irradiated hybrid cells, and further challenged with live P815 cells harvested from ascites were killed 3 months after the last treatment. 6x10⁶ splenocytes were stimulated with 10⁵ irradiated (15 000 rads) P815 in a volume of 2 ml of DMEM containing additives and 2% ultroser HY. After 5 days of culture, the effectors generated were tested for lytic activity in a 3.5-h ⁵¹Cr-release assay on P815. 50 *µ*l of supernatants were collected after 24 h of culture, frozen and assayed for IL-2 content. IL-2 production by cells from the peritoneal cavity was tested as follows: the cells were harvested from the same treated mice by extensive washing of the peritoneal cavity with cold DMEM, and 6x10⁴ peritoneal exudate cells were cultured (in DMEM containing 1% mouse serum and additives) with various numbers of irradiated P815 cells in round-bottom 96-well plates. The supernatants were collected after 48h of culture and assayed for IL-2 content.

### In vivo treatments

Cultured tumor cells were washed three times with PBS and resuspended in PBS for implantation into mice. DBA/2 mice were injected intraperitoneally with 2x10⁵ P815 or 2x10⁴ L1210 tumor cells. Some animals received 3 or 7 injections of 2x10⁶ irradiated P815 tumor cells or hybrid cells, cultured or not with GM-CSF, every 5 days starting on day 3 after tumor inoculation. In the experiment 2x10⁵ P815 cells were injected intraperitoneally into sublethally irradiated DBA/2 mice (800 rads) and tumor cells harvested from ascites were used to assess tumor resistance in vivo.

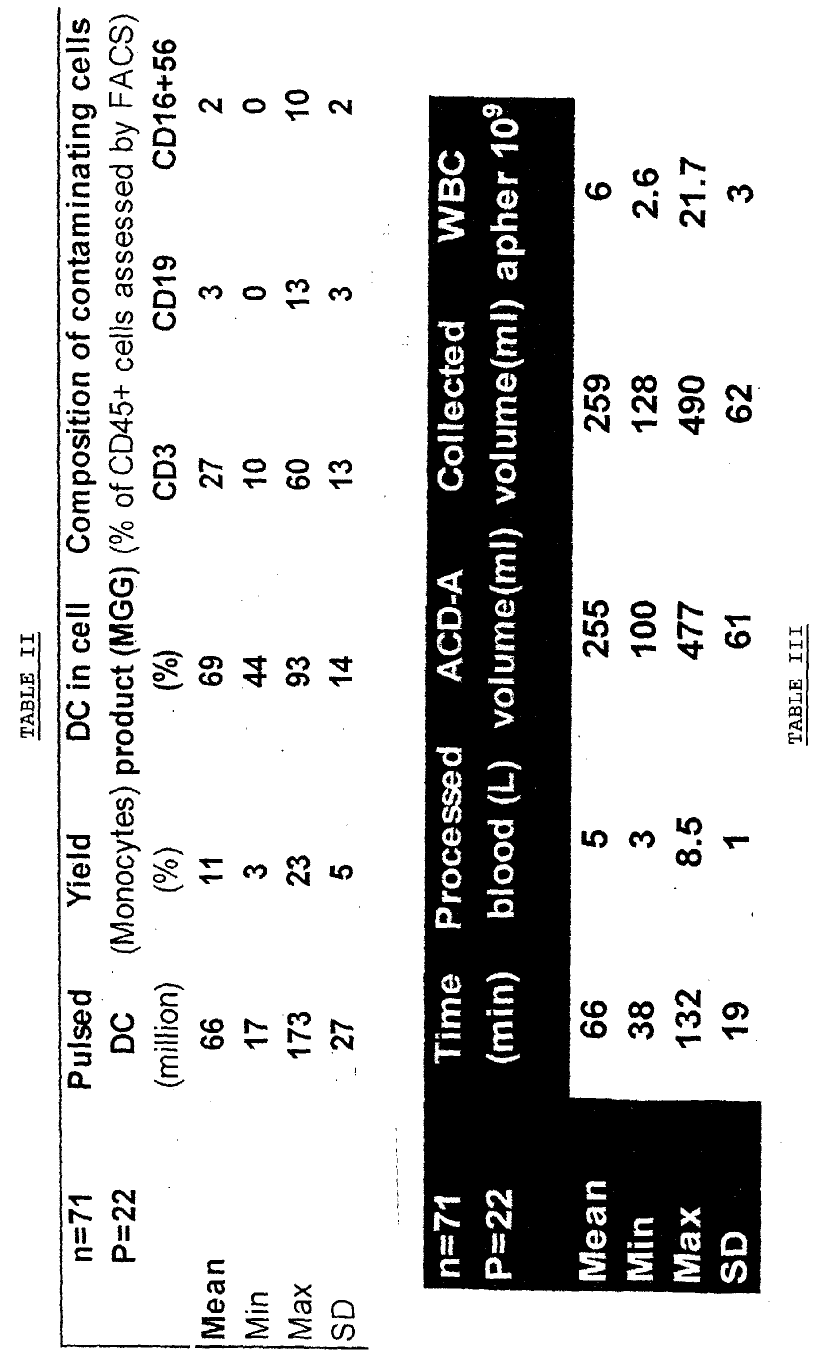

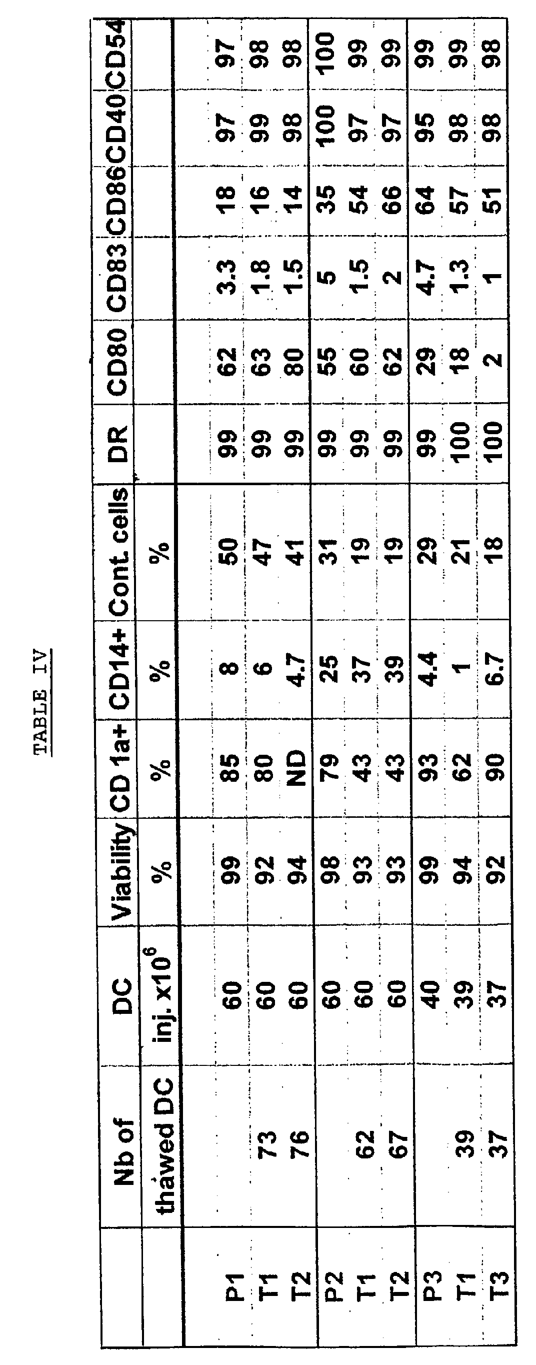

### REFERENCES

1. Steinman RM, *Annu Rev Immunol* 1991; 9:271-296
2. Schuler G et al., *J Exp Med* 1997; 18: 1183-1187
3. Santiago-Schwarz F et al., *J. Leukoc Biol* 1992; 52:274-281
4. Sallusto F and Lanzavecchia A., *J Exp Med* 1994; 179:1109-1118
5. Siena S et al., *Exp Hematol* 1995; 23:14631471
6. Hsu FJ et al., *Nat Med* 1996; 2:52-58
7. Nestle FO et al., *Nat Med* 1998; 4:328-332
8. Murphy G et al., *The Prostate* 1996; 29:371-380
9. Thurner B et al., *J Immunol Methods* 1999; 223: 1-15
10. Foy TM et al., *Annu Rev Immunol* 1996 ; 14 :591-617
11. Cella M et al., *Nature* 1997; 388:782-7
12. Rieser C et al., *Blood* 1998; 91:3112-3117
13. Lethé et al., *European Journal of Immunology* 1992; 22:2283-2288

## Claims

1. Composition containing blood cells, characterised in that it comprises a percentage of dendritic cells higher than 30% and lower than 95% of the total number of blood cells present in the composition.

2. Composition according to claim 1, characterised in that the percentage of dendritic cells is higher than 50%, preferably higher than 60%, and lower than 90% of the total number of blood cells present in the composition.

3. Composition according to claim 1 or 2, characterised in that the percentage of dendritic cells is higher than 70% and lower than 90% of the total number of blood cells present in the composition.

4. Composition according to any one of the preceding claims, characterised in that it comprises lymphocytes, preferably lymphocytes T (CD3+).

5. Composition according to any one of the preceding claims, characterised in that the dendritic cells are immature autologous clinical grade dendritic celles or are dendritic-like cells/tumour cells hybrids or hybridomas, preferably dendritic cells/tumour cells hybrids and hybridomas.

6. Composition according to any one of the preceding claims, characterised in that it is included in a closed system, preferably in a sterile batch.

7. Composition according to any one of the preceding claims, characterised in that the blood cells are pulsed with tumour-associated antigens (TAA).

8. Pharmaceutical composition comprising a suitable pharmaceutical carrier or diluent and the composition according to any one of the preceding claims.

9. Use of the pharmaceutical composition according to claim 8, for the manufacture of a medicament intended for inducing an anti-tumour immune response in a patient, especially for the treatment and/or the prevention of carcinogenesis and/or cancer, including tumour development and metastases formation.

10. Method for the generation of immature autologous clinical grade dendritic cells in a closed system, comprising the step of:
- generating said cells in the close system from leukapheresis products by using clinical grade cytokines which are thereafter harvested for several days (preferably 7 days) culture and loaded with relevant clinical grade of tumour-associated antigens (TAA) or loaded with a tumoral cell obtained from the patient for the generation of one or more dendritic-like cells / tumour cells hybrids or hybridomas.

11. Method according to claim 10, wherein the cytokines are IL-4 and GM-CSF or other molecules having similar properties upon the maturation and/or the differentiation of monocytes.

12. Method according to claim 10 or 11, characterised in that the leukapheresis products are submitted to one or more centrifugation steps in order to obtain platelet rich plasma (PRP) which is added with a synthetic culture medium comprising X-VIVO 20 in a culture bag in order to allow adherence of monocyte cells present in said leukapheresis products for about 1 hour.
